# EUROPÄISCHE PATENTANMELDUNG

(11) **EP 1 559 416 A2**
(43) Veröffentlichungstag der Anmeldung: **03.08.2005**
(21) Anmeldenummer: 04106774.5
(22) Anmeldetag: 21.12.2004
(51) Int. Cl.: A61K 7/48, A61K 7/00, A61K 7/42

(54) **Dünnflüssige O/W-Emulsion I**

(30) Priorität: 21.01.2004 DE 102004003437
(71) Anmelder: Beiersdorf Aktiengesellschaft, D-20253 Hamburg (DE)
(72) Erfinder: Nielsen, Jens, 24558 Henstedt-Ulzburg (DE); Raschke, Thomas, 25421 Pinneberg (DE); Voigt, Nadine, 22455 Hamburg (DE)

(57) **Zusammenfassung**

Kosmetische und/oder dermatologische Öl-in-Wasser-(O/W-) Emulsion mit einer Viskosität von 100 bis 1500 mPaS, enthaltend
a) 0,5 bis 8 Gewichts-% eines oder mehrerer Elektrolyten,
b) 0,05 bis 1 Gewichts-% Verdicker,
c) c) 0,5 bis 5 Gewichts-% , Glycerylstearatcitrat
jeweils bezogen auf das Gesamtgewicht der Zubereitung,
wobei der Zubereitung keine Fettalkohole zugesetzt sind.

## Beschreibung

Die vorliegende Erfindung betrifft eine dünnflüssige Öl-in-Wasser- (O/W-) Emulsion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV -Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Cremes, Salben oder Lotionen, dienen meist der Befeuchtung und Rückfettung der Haut sowie der Versorgung der Haut mit kosmetischen oder dermatologischen Wirkstoffen (z.B. Vitaminen, Antioxidantien, UV-Lichtschutzfiltern). Dies ist gerade dann wichtig, wenn das natürliche Regenerationsvermögen der Haut nicht ausreicht, um die durch Umwelteinflüsse, mechanische oder chemische Reize, Sonnenlicht und Wind entstandenen Belastungen (die u.a. die Hautalterung beschleunigen) und Schäden der Haut auszugleichen.

Eine besonders weit verbreitete Form kosmetischer Emulsionen stellen dünnflüssige Öl-in-Wasser-Emulsionen (O/W-Emulsionen) dar. Dünnflüssige O/W-Emulsionen basieren in der Regel auf einem Emulgatorsystem aus Ceteareth-20, Ceteareth 12, Cetearylalkohol, Cetylpalmitate und Glycerylstearat. Weitere O/W-Emulgatorsysteme sind: Triceteareth-4 Phosphate; Trilaureth-4 Phosphate; Glyceryl Isostearate + lsoceteth-20; PEG-60 Hydrogenated Castor Oil oder Polysorbate 20.

Derartige Zubereitungen des Standes der Technik haben jedoch den Nachteil, dass sie, nicht zuletzt aufgrund ihres relativ hohen Emulgatorgehaltes, ein klebriges Hautgefühl aufweisen und wenig Pflege bieten. Ferner weisen derartige Zubereitungen in der Regel keine allzu hohe Lagerstabilität (Langzeitstabilität) auf.

Außerdem sind nach dem Stande der Technik hochviskose O/W-Emulsionen mit Polyethylenglykolestern als Emulgatoren bekannt. Emulsionen mit Polyethylenglykolestern als Emulgatoren haben den Vorteil, dass sie langzeitstabil und elektrolytkompatibel sind. Ferner besitzen sie ein reichhaltiges, nicht klebriges Hautgefühl und weisen eine gute Hautverträglichkeit auf.

Derartige Zubereitungen haben jedoch den Nachteil, dass sie aufgrund Ihrer Zähflüssigkeit nur ein beschränktes Anwendungsspektrum abdecken. Sie sind weder versprühbar noch eignen sie sich als Tränkmedium für Tücher.

Um die Probleme des Standes der Technik zu lösen wurden in der Vergangenheit unterschiedliche Lösungswege beschritten. Beispielsweise wurden Pickering-Pigmente als Emulgatoren eingesetzt (DE 102 38 649.8). Ein weiterer Lösungsansatz bestand darin, eine hohe Konzentration an Emulgator in Kombination mit Fettalkoholen einzusetzen (DE 198 02 204-6 und DE 101 41 258 sowie EP 1074242, EP 1077062 und EP835651) oder W/O-Emulsionen zu verwenden (DE 101 54 627). Fettalkohole haben jedoch den Nachteil, dass sie die Viskosität von O/W- Emulsionen stark erhöhen (auch wenn sie die Stabilität von O/W-Emulsionen verbessern) und das Hautgefühl sehr stark beeinflussen. Eine Stabilitätserhöhumg der Emulsion erkauft man sich somit auf Kosten der sensorischen Flexibilität.

O/W-Emulsionen des Standes der Technik haben ferner den Nachteil, eine nur geringe Temperaturstabilität bei extremen Temperaturen und in einem größeren Temperaturintervall (-10 C° bis 40 C°) aufweisen. Insbesondere UV-Lichtschutzzubereitungen müssen jedoch sowohl im Sommer (z.B. am Strand) als auch im Winter (z.B. beim Ski fahren) hohen thermischen Belastungen standhalten.

Ferner lassen sich insbesondere in dünnflüssige O/W-Emulsionen des Standes der Technik nur relativ gering Mengen an Elektrolyten einarbeiten. insbesondere Elektrolytgehalte von mehr als 1 Gewichs-% neigen (insbesondere bei thermischer Beanspruchung) zur Phasentrennung.

Es war deshalb die Aufgabe der vorliegenden Erfindung, dünnflüssige O/W-Emulsionen zu entwickeln, welche die Mängel des Standes der Technik vermeiden.

Ferner war es die Aufgabe der vorliegenden Erfindung, dünnflüssige, besonders lagerstabile O/W-Emulsionen zu entwickeln, die sich durch ein allenfalls minimal klebriges Hautgefühl aufweisen und besonders hautverträglich sind. Weiterhin war es die Aufgabe der vorliegenden Erfindung eine besonders temperaturstabile dünnflüssige O/W-Emulsion zu entwickeln, die auch noch bei hohen Elektrolytgehalten über einen längeren Zeitraum temperatur- und lagerstabil ist.

Nicht zuletzt wurde die Anforderung an die O/W-Emulsion gestellt, sowohl als Tränkzubereitung für Tücher und andere Textilien geeignet zu sein, als auch mittels einer Sprühvorrichtung (z.B. Aerosoldose, Pumpspray) versprühbar zu sein.

Überraschend gelöst werden die Aufgaben durch eine kosmetische und/oder dermatologische Öl-in-Wasser-(O/W-) Emulsion mit einer Viskosität von 100 bis 1500 mPaS, enthaltend
a) 0,5 bis 8 Gewichts-% eines oder mehrerer Elektrolyten,
b) 0,05 bis 1 Gewichts-% Verdicker,
c) 0,5 bis 5 Gewichts-% , Glycerylstearatcitrat
jeweils bezogen auf das Gesamtgewicht der Zubereitung,
wobei der Zubereitung keine Fettalkohole zugesetzt sind.

Bei den erfindungsgemäßen O/W-Emulsionen handelt es sich um überraschend stabile, dünnflüssige Emulsionen, die hohe Mengen an Elektrolyten enthalten und ein hervorragendes Hautgefühl aufweisen. Die erfindungsgemäßen Emulsionen sind bei Lagerung bei RT oder 40 °C über 3 Monate stabil.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur: 20°C, Spindeldurchmesser 24 mm, Rotorgeschwindigkeit 62 1/min).

Erfindungsgemäß bevorzugte Ausführungsformen der vorliegenden Erfindung sind dadurch gekennzeichnet, dass Glycerylstearatcitrat in einer Konzentration von 0,5 bis 4 Gewichts-% und besonders bevorzugt in einer Konzentration von 1 bis 3 Gewichts-%, jeweils bezogen auf das Gesamtgewicht der Zubereitung, eingesetzt wird.

Glycerylstearatcitrat, (2-Hydroxy-1,2,3-propantricarbonsäure-1,2,3-propantriolmonoocta-decanoat, INCI Glyceryl Stearate Citrate, CAS 39175-72-9) ist der Citronensäureester des Glycerylstearat und u.a. unter der Bezeichnung Imwitor 370 bei der Firma Hüls im Handel erhältlich.

Erfindungsgemäß bedeutet "keine Fettalkohole zugesetzt", das der erfindungsgemäßen Zubereitung keine Fettalkohole zugesetzt werden, wobei aber auch solche Zubereitungen als erfindungsgemäß anzusehen sind, bei denen Spuren von Fettalkoholen in der Zubereitung vorhanden sind, wenn diese in Form von Verunreinigungen anderer Inhaltsstoffe der Zubereitung in diese eingeschleppt worden sind. Erfindungsgemäß bevorzugt ist die erfindungsgemäße Emulsion frei von Fettalkoholen.

Darüber hinaus kann die erfindungsgemäße Emulsion weitere Emulgatoren enthalten, die aus der Liste der aus der Kosmetik bekannten Emulgatoren gewählt werden können.

Bevorzugte Ausführungsformen der vorliegenden Erfindung enthalten als Verdicker eine oder mehrere Verbindungen gewählt aus der Gruppe Carrageenan, Xanthangummi, Polyacrylate, Hydroxypropylmethylcellulose, Celluloseether, AcryloyldimethyltaurateNP Copolymer, Hydroxypropyldistärkephosphat (CAS Nummer 113894-92-1).

Das erfindungsgemäße Carrageenan (INCI Chondrus Crispus (Carrageenan), CAS 9000-07-1) ist u.a. bei der Firma FMC unter dem Handelsnamen Gelcarin erhältlich. Erfindungsgemäß vorteilhaft wird Carragenan in einer Konzentration von 0,1 bis 2 Gewichts-% und besonders bevorzugt in einer Konzentration von 0,2 bis 1 Gewichts-%, jeweils bezogen auf das Gesamtgewicht der Emulsion eingesetzt.

Das erfindungsgemäße Xanthangummi (INCI Xanthan Gum, CAS 11138-66-2) ist u.a. bei der Firma Kelco unter dem Handelsnamen Keltrol F erhältlich. Erfindungsgemäß vorteilhaft wird Xanthangummi in einer Konzentration von 0,01 bis 2 Gewichts-% und besonders bevorzugt in einer Konzentration von 0,05 bis 0,5 Gewichts -%, jeweils bezogen auf das Gesamtgewicht der Emulsion eingesetzt.

Als erfindungsgemäße Polyacrylate können erfindungsgemäß vorteilhaft Homo- oder Copolymere der Arcrylsäure und/oder ihrer Derivate mit der folgenden Struktur eingesetzt: 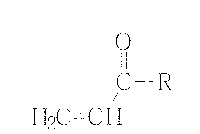 mit R= OH, OR', NHR', mit R'= Alkyl-, Aryl-, Heteroalkyl-, Heteroaryl- Alkylsulfonate, beispielsweise mit der folgenden Struktur:

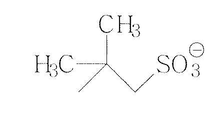

Als erfindungsgemäß vorteilhafte Comonomere der erfindungsgemäß vorteilhaften Copolymere können beispielsweise eingesetzt werden: C₁₀-C₃₀Alkylacrylate, Methacrylsäure und Acrylamid.

Erfindungsgemäß bevorzugt ist der Einsatz der Homopolymere der Acrylsäure. Besonders bevorzugt weisen diese ein Molekulargewicht von 2000000 bis 6000000 auf wie z.B. die Handelsprodukte Pemulen® Typen TR 1, TR 2 und TRZ, Pas 980, 981, 984 1342, 1382, 2984 und 5984. ebenso die ETD-Typen 2001, 2020, 2050 und Carbopol Ultrez 10 der Firma Noveon sowie das Stabileze 06 der Firma ISP

Hydroxypropylmethylcellulose (CAS 9004-65-3) ist u.a. bei der Firma Dow Chemical unter dem Handelsnamen Methocel 4EM erhältlich. Erfindungsgemäß vorteilhaft wird es in einer Konzentration von 0,05 bis 2 Gewichts-% und besonders bevorzugt in einer Konzentration von 0,2 bis 1 Gewichts-%, jeweils bezogen auf das Gesamtgewicht der Emulsion eingesetzt.

Eine besonders bevorzugte Ausführungsform der vorliegenden Erfindung stellen Emulsionen dar, die als Emulgator Glycerylstearatcitrat in einer Konzentration von 0,5 bis 5 Gewichts-% und als Verdicker eine Kombination aus Carrageenan und Xanthangummi im Verhältnis von 1:5 bis 50 :1 enthalten.

Als Elektrolyten werden erfindungsgemäß Verbindungen verstanden, die in Form wasserlöslicher Salze vorliegen. Die Kationen und Anionen der Elektrolyte können sowohl anorganische als auch organisch-chemische Ionen darstellen. Insbesondere können die Salze aus Anionen anorganischer Mineralsäuren (z.B. Salzsäure, Salpetersäure, Schwefelsäure, Kohlensäure, Phosphorsäure) und/oder organischer Carbonsäuren (auch Milchsäure, Zitronensäure) und Sulfonsäuren gebildet werden.

Als Kationen werden meist, wenn auch nicht ausscchließlich, anionische Kationen z.B. Natrium-, Kalium-, Calcium-, Magnesiumionen eingesetzt.

Als erfindungsgemäß bevorzugte Salze werden Natriumchlorid , Ma gnesiumchlorid, Kaliumchlorid, Taurin, Citronensäure, Milchsäure, Ascorbinsäure, Kreatin, Salicylsäure, Carnitin sowie UV-Filtersubstanzen gewählt aus der Gruppe Phenylbenzimidazolsulfonsäure, Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, insbesondere das Phenylen-1,4-bis-(2-benzimidazyl)-3,3'-5,5'-tetrasulfonsäure-bis-natriumsalz, Terephthalidendicamphersulfon-säure oder ihre Salze, Salze der 2-Phenylbenzimidazol-5-sulfonsäure, wie ihr Natrium-, Kalium- oder ihr Triethanolammonium-Salz, sowie die Sulfonsäure selbst; und/oder Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze eingesetzt.

Erfindungsgemäß vorteilhaft enthält die erfindungsgemäße Emulsion kosmetische und/oder dermatologische Wirkstoffe. Insbesondere ist es erfindungsgemäß bevorzugt, wenn die Emulsion UV-Lichtschutzfilter und/oder kosmetische Wirkstoffe enthält.

Die erfindungsgemäßen Emulsionen können als wässrige Phase neben Wasser erfindungsgemäß vorteilhaft auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, Methylpropandiol, 1,2-Propandiol und Glycerin.

Die erfindungsgemäße Emulsion enthält ein oder mehrere Öl-, Lipid-, und/oder Wachskomponenten gleicher oder unterschiedlicher Polarität. Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.
Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

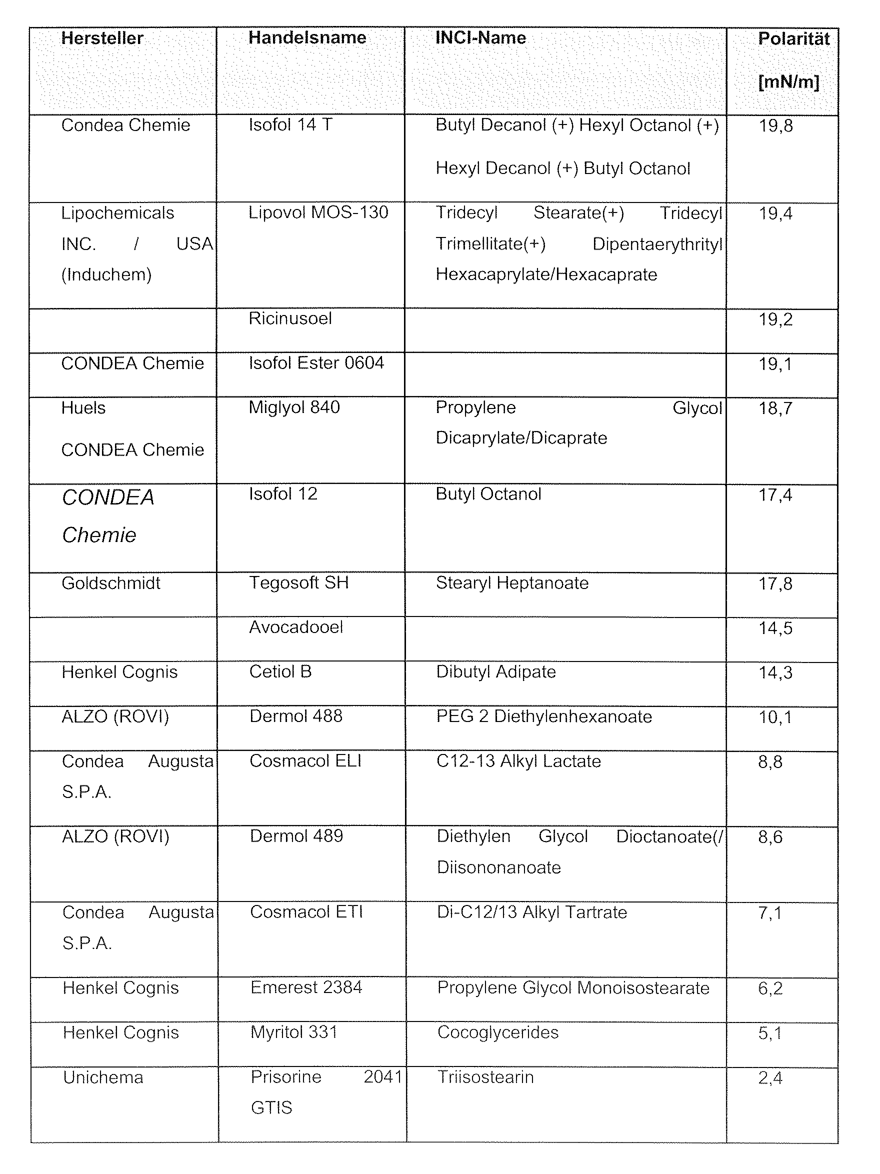

Ferner kann die Ölphase vorteilhaft gewählt werden aus der Gruppe der Dialkylether. Es ist insbesondere vorteilhaft, wenn die Ölphase einen Gehalt an C₁₂₋₁₅ -Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 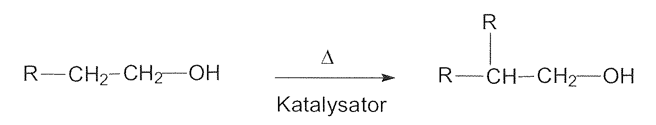 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 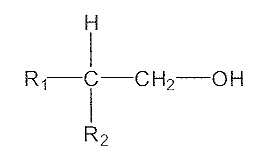 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
- R₁ =: Propyl, Butyl, Pentyl, Hexyl, Heptyl oder Octyl und
- R₂ =: Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 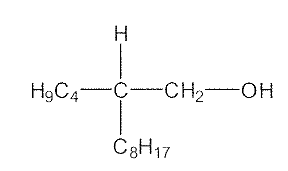 und ist beispielsweise unter der Handelsbezeichnung Isofol® 12 von der Gesellschaft Condea Chemie GmbH erhältlich - und das 2-Hexyldecanol - es hat die chemische Struktur 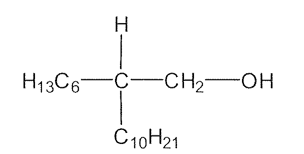 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

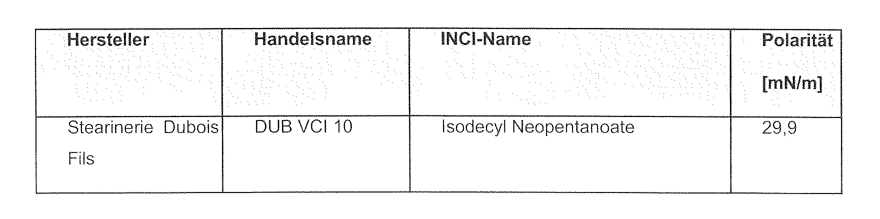

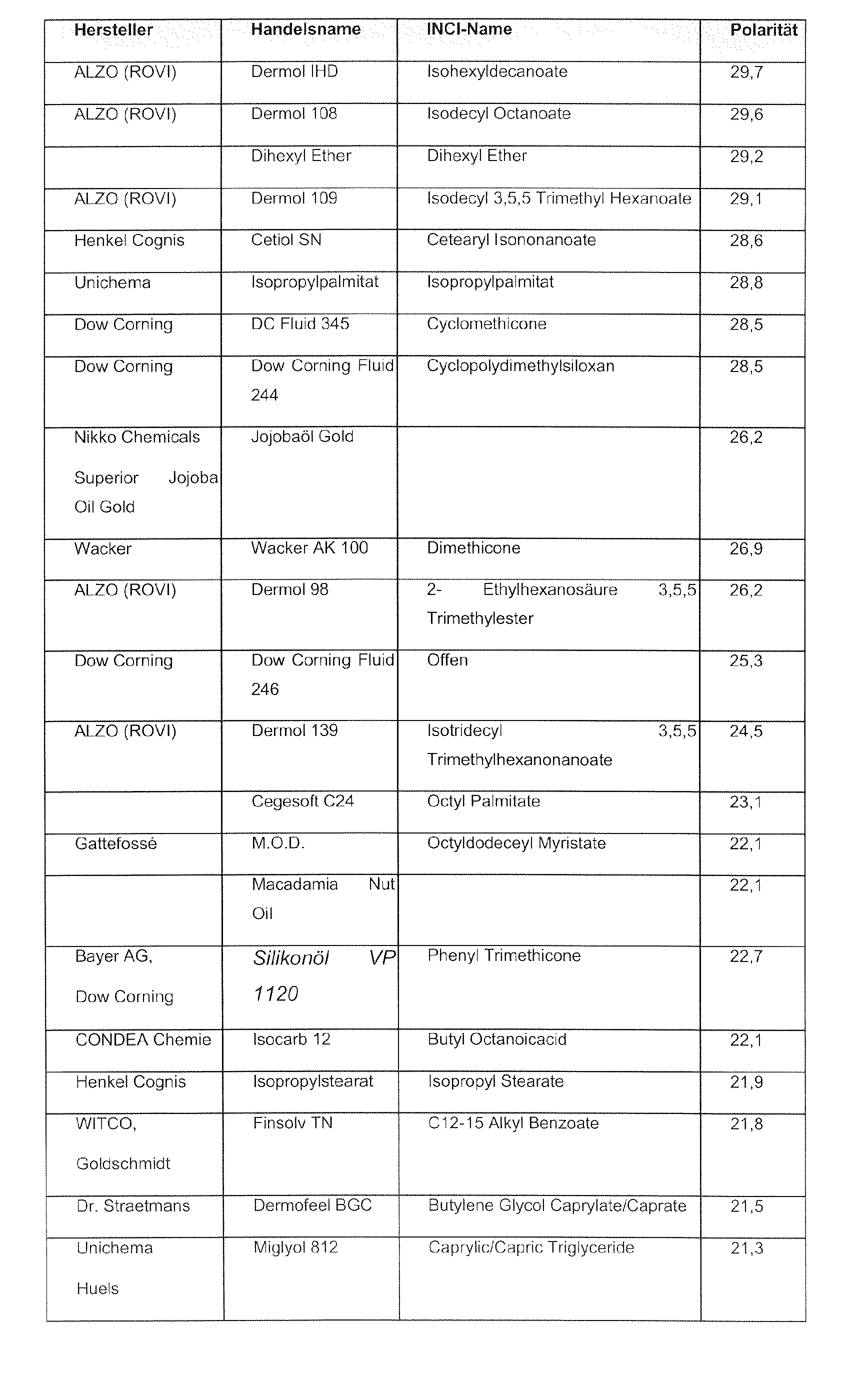

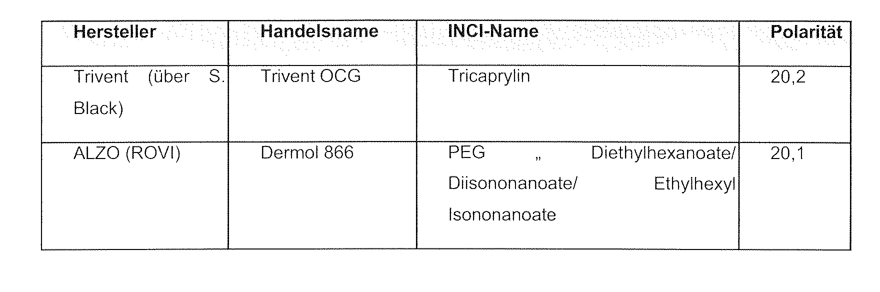

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

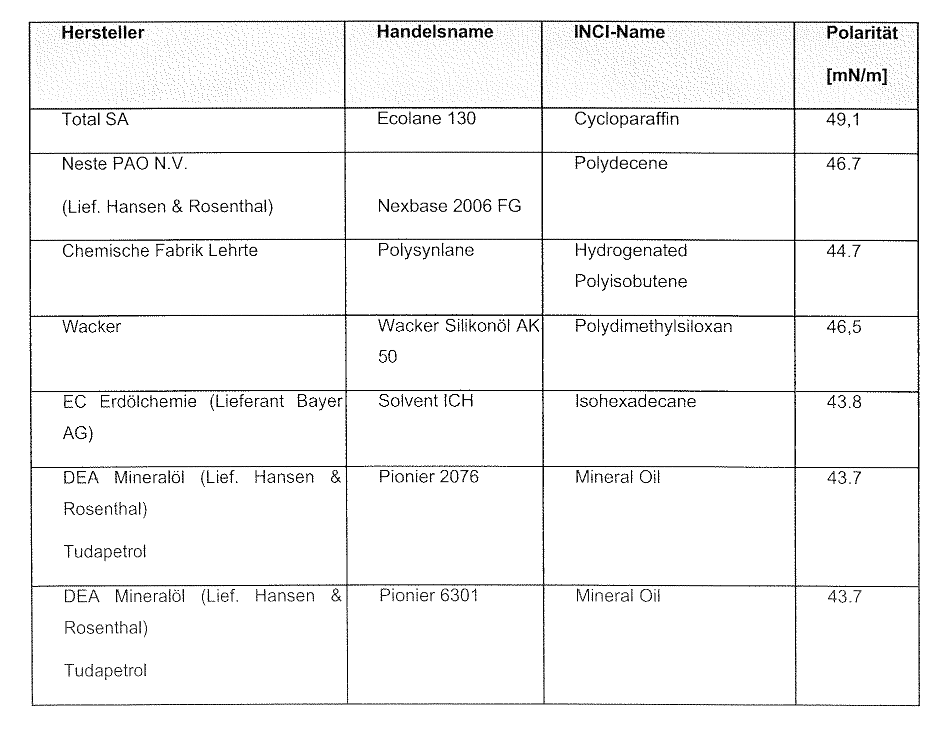

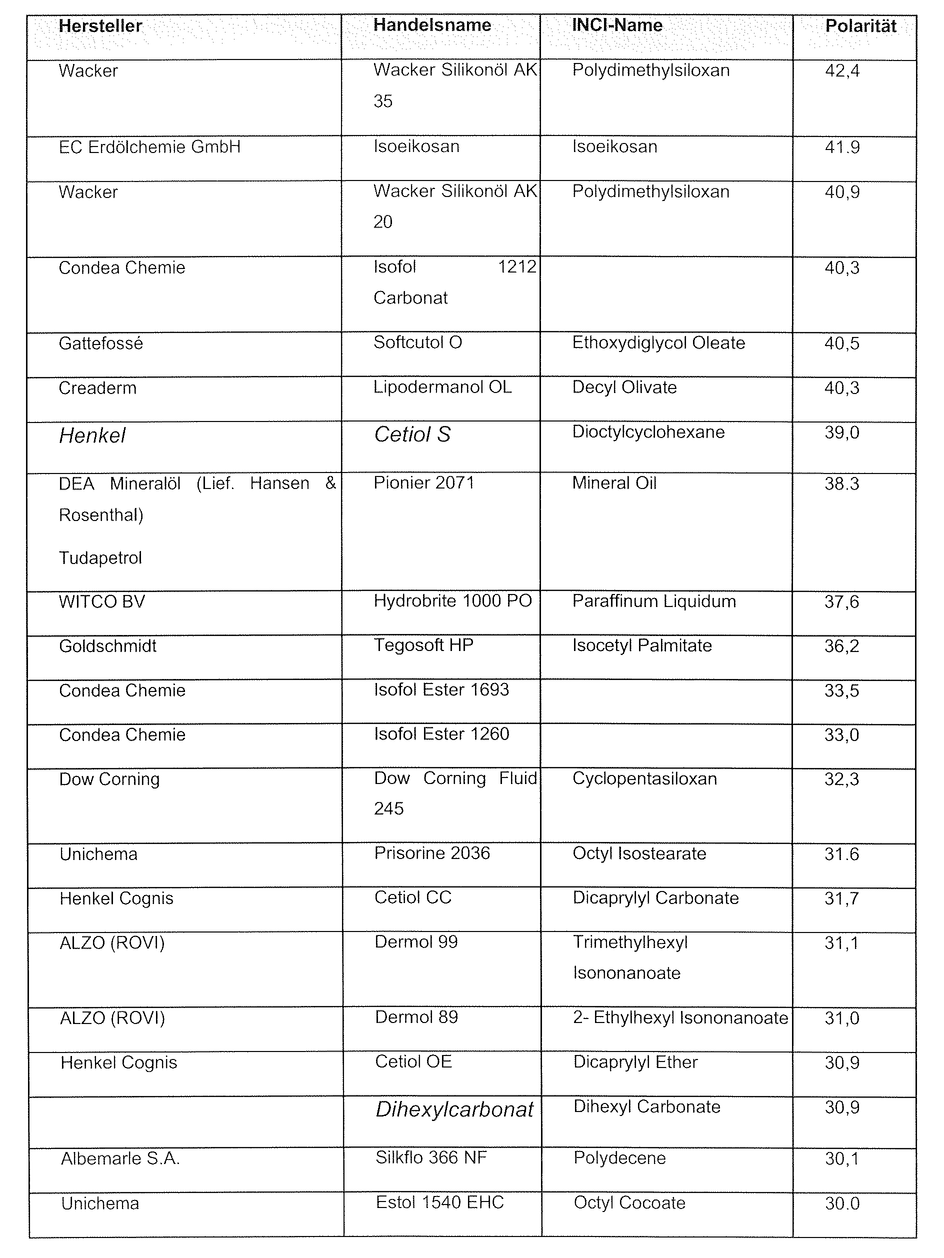

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

### Silikone

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

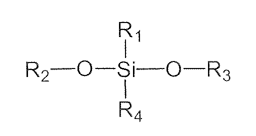

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 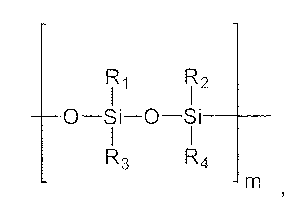 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 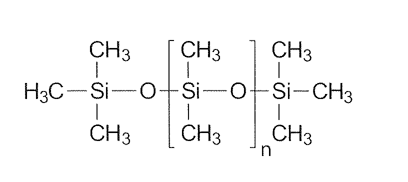 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (iNCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

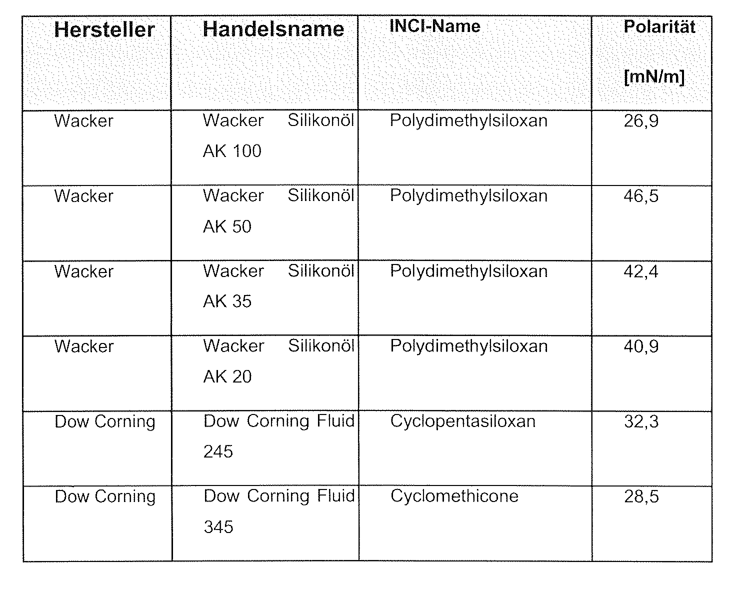

Erfindungsgemäß vorteilhaft einzusetzende cyclische Silicone werden im allgemeinen durch Strukturelemente charakterisiert, wie folgt 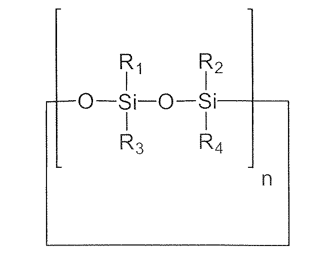 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Die erfindungsgemäßen Emulsionen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Erfindungsgemäß bevorzugt enthalten die erfindungsgemäßen Emulsionen ein oder mehrere Antioxidantien. Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbindung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid -Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptidund/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

Weitere vorteilhafte Wirkstoffe im Sinne der vorliegenden Erfindung sind natürliche Wirkstoffe und/oder deren Derivate, wie z. B. alpha-Liponsäure, Phytoen, Lycopene D-Biotin, Coenzym Q10, Carnitin, Vitamin A und seine Derivate, natürliche und/oder synthetische Isoflavonoide (z.B. Genistein, Daidzein), Kreatin, Kreatinin, Taurin und/oder ß-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Niacinamid, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel. Auch die Vitamin D₃-analoga Tacalcitol, Calcipotriol, Tacalcitol, Colecalciferol sowie Calcitrol (Vitamin D₃) und/oder Fumarsäureester können erfolgreich in die Zubereitungen eingearbeitet werden.

Die erfindungsgemäßen Zubereitungen können einen oder mehrere dieser Wirkstoffe (Moisturizer, Antioxidantien, sonstige Wirkstoffe) jeweils in einer Konzentration von 0,001 bis 30 Gewichts-%, bezogen auf das Gesamtgewicht der Zubereitung enthalten.

Es ist erfindungsgemäß vorteilhaft, wenn die erfindungsgemäßen Emulsionen einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Emulsionen im Sinne der vorliegenden Erfindung vorzugsweise mindestens eine UV-A- und/oder UV-B-Filtersubstanz. Neben den zu den Elektrolyten zu zählenden UV-Filtersubstanzen können weitere Lichtschutzfilter eingesetzt werden. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂) Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein. Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure - | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), ), welches von Givaudan unter der Marke Parsol® 1789 und von Merck unter der Handelsbezeichnung Eusolex® 9020 verkauft wird.

Weitere vorteilhafte UV-A-Filtersubstanzen sind Hydroxybenzophenone, die sich durch die folgende Strukturformel auszeichnen: 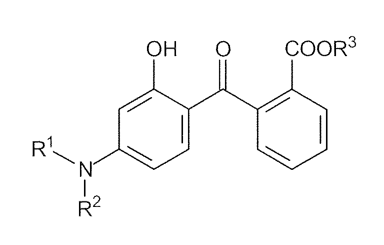 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 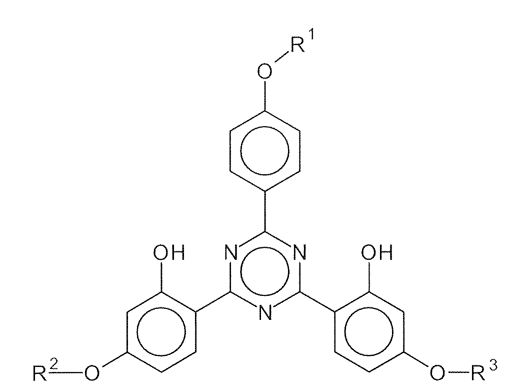 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)] und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 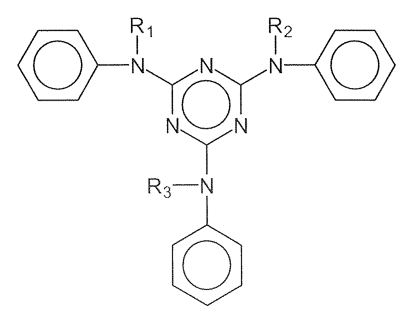 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 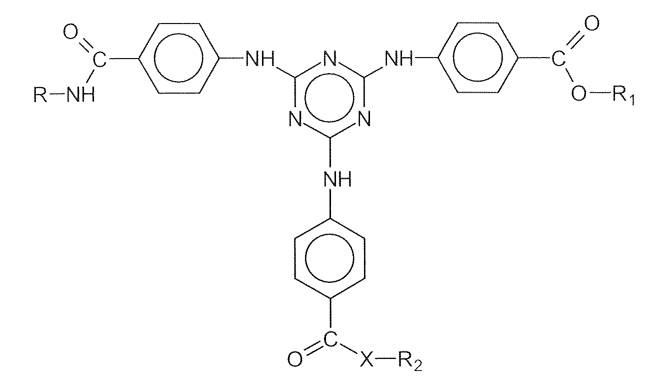 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Al kylgruppen, oder ein Wasserstoffatom, ein Alkalimetallatom, eine Ammoniumgruppe oder eine Gruppe der Formel 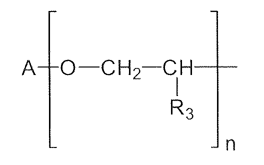 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 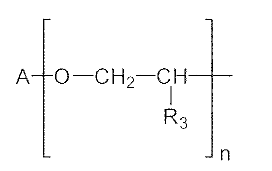 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt, wenn X ein Sauerstoffatom darstellt.

Besonders bevorzugte UV-Filtersubstanz im Sinne der vorliegenden Erfindung ist ferner ein unsymmetrisch substituiertes s-Triazin, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 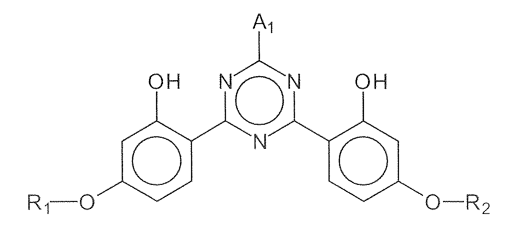 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methylpyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Um die Wasserfestigkeit kosmetischer und dermatologischer Lichtschutzzubereitungen zu verbessern, kann es von Vorteil sein, Filmbildner in die kosmetischen oder dermatologischen Zubereitungen einzuarbeiten. Nach dem Stand der Technik eignen sich hierzu z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), C₂₀₋₄₀ Carbonsäure mit Polyethylen (Performacid 350 von der Fa. New Phase Technologies) sowie Filmbildner aus der Gruppe der Polymere auf Basis von Polyvinylpyrrolidon (PVP):

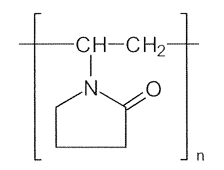

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Erfindungsgemäß vorteilhaft kann die erfindungsgemäße Emulsion Mikropartikel enthalten, deren mittlerer Partikeldurchmesser zwischen 1 nm und 200 nm, besonders vorteilhaft zwischen 5 nm und 100 nm liegt.

Es ist ferner vorteilhaft, die Konzentration aller Partikel größer als 0,1 Gew.-%, besonders vorteilhaft zwischen 0,1 Gew.-% und 30 Gew.-%, bezogen auf das Gesamtgewicht der Zubereitungen, zu wählen.

Im wesentlichen unerheblich für die vorliegende Erfindung ist es, in welcher der gegebenenfalls natürlich vorkommenden Modifikationen die Partikel vorliegen.

Vorzugsweise werden unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Vorteilhaft sind ferner Pigmente, die oberflächlich wasserabweisend behandelt ("gecoatet") sind. Diese Oberflächenbehandlung kann darin bestehen, daß die Pigmente nach an sich bekannten Verfahren mit einer dünnen hydrophoben Schicht versehen werden.

Besonders vorteilhaft sind TiO₂-Pigmente, beispielsweise die mit Aluminiumstearat beschichteten, unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise sind ferner Bornitridpartikel, beispielsweise die im folgenden aufgelisteten Bornitride:

| **Handelsname** | **erhältlich bei** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Vorteilhaft sind ferner beispielsweise die bei der Firma Carborundum unter der Handelsbezeichnung Très BN® UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Weitere vorteilhafte Pigmente sind mikrofeine Polymerpartikel.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen.

Dabei ist es im wesentlichen unerheblich, in welcher Form (Plättchen, Stäbchen, Kügelchen etc.) die verwendeten Polymerpartikel vorliegen.

Desweiteren ist es vorteilhaft, modifizierte Polysaccharide zu verwenden.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der HydroxyGruppen der Polyglucane durch Veretherung, Veresterung oder selektive Oxidation. Dabei entstehen z. B. sogenannte Stärkeether und Stärkeester der allgemeinen Strukturformel 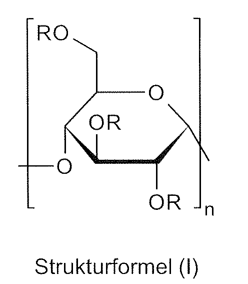 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet 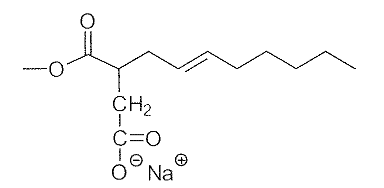 und welches z. B. unter der Handelsbezeichnung Amiogum® 23 bei der Firma CERESTAR erhältlich ist sowie Aluminium Stärke Octenylsuccinate, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Tenside, Pigmente, die eine färbende Wirkung haben, Verdickungsmittel, weich machende, anfeuchtende und/oder feucht haltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5 -Chlor-2-methyl-4-isothiazolin-3-on, 2 -Chloracetamid, Benzalkoniumchlorid, Benzylalkohol.
Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Eine besonders bevorzugte Ausführungsform der vorliegenden Erfindung stellen Textilien dar, die mit einer erfindungsgemäßen Emulsion getränkt sind. Dabei ist es erfindungsgemäß bevorzugt, wenn das Textil in Form eines ungewebten Vlieses, insbesondere in Form eines Tuches oder "Pads", vorliegt.

Erfindungsgemäß bevorzugt ist ein Textil, welches aus einer Mischung von Viskosefasern, Polyesterfasern und/oder Baumwollfasern gebildet wird.

Vorteilhaft im Sinne der vorliegenden Erfindung ist es insbesondere, wenn der Textilstoff des erfindungsgemäßen Textils gebildet wird aus
1-30 Gewichts-% Baumwollfasern,
9-80 Gewichts-% Viskosefasern und
19-90 Gewichts-% Polyester,
jeweils bezogen auf das Gesamtgewicht des Textils.

Ganz besonders bevorzugt weist das erfindungsgemäße Textil an der Oberfläche einen Baumwollanteil bis 30 Gewichts-% und im Inneren einen Baumwollanteil bis 10 Gewichts-%, jeweils bezogen auf das Gesamtgewicht des Textils, auf.

Erfindungsgemäß vorteilhaft ist insbesondere der Einsatz der erfindungsgemäßen Emulsion als sprühbarer Emulsion. In einem solchen Falle kann die erfindungsgemäße Emulsion mit Hilfe eines Druckgases aus einer Aerosoldose oder mit Hilfe einer Pumpe aus einem Pumpspray-Applikator (auch Pumpzerstäuber) heraus angewendet werden.

Erfindungsgemäß ist auch ein Sprayapplikator (Sprüh-Applikator) in Form eines Pumpsprayapplikators oder einer Aerosoldose enthaltend eine Emulsion nach einem der vorhergehenden Ansprüche.

Erfindungsgemäß ist die Verwendung der erfindungsgemäßen Emulsion oder eines Textils oder Sprüh-Applikator zur Pflege der Haut, insbesondere der Gesichtshaut, der Altershaut, trockener oder anderweitig beanspruchter Haut und insbesondere zum Schutz der Haut vor UV-Strahlung.

| **Vergleichsversuch:** | | |
|---|---|---|
| | O/W-Emulsion 5 mit 2,5 % NaCl | O/W-Emulsion 5 + 2% Cetearylalkohol mit 2,5 % NaCl |
| Viskosität | 1000 mPas | 2000 mPas |
| Versprühbarkeit | Ja | Nein |

Versprühbarkeit bedeutet, dass die Emulsion aus einem Packmittel, wie man sie bei Lichtschutzsprays verwendet, versprüht werden kann. Die Emulsion wird dabei durch Betätigung (Herunterdrücken) des Sprühkopfes mit dem Zeigefinger versprüht, wobei die Emulsion nicht als einzelner Strahl sondern möglicht feindispers versprüht werden soll um eine gute Verteilung auf der Haut zu ermöglichen.
Ein ja bedeutet feindisperses Versprühen. Ein nein bedeutet, dass die Emulsion gar nicht oder nur als einzelner Strahl versprüht werden kann. Letzteres wäre für eine praktische Anwendung inakzeptabel.
Der Vergleichsversuch zeigt, dass durch den erfindungsgemäßen Verzicht auf Fettalkohole, die erfindungsgemäße Zubereitung versprühbar wird.

Die folgenden Beispiele, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispiele

### (1) O/W Emulsion

| | Gew.-% |
|---|---|
| Glycerylstearatcitrat | 1,50 |
| Jojobaöl | 2,00 |
| Caprylic/Capric Triglycerid | 1,00 |
| Dicaprylylcarbonat | 2,00 |
| Vaseline | 1,00 |
| Dimethicon (linear) | 2,00 |
| Carageenan | 0,20 |
| Xanthan Gum | 0,10 |
| Ethylhexylcyanodiphenyl-acrylat (Octocrylen) | 3,00 |
| Bis-Ethylhexyloxyphenol- | 1,00 |
| methoxyphenyltriazine | |
| Phenylbenzimidazol-sulfonsäure | 2,00 |
| Natriumascorbylphosphat | 0,10 |
| Tocopherylacetat | 1,00 |
| Glycerin | 7,00 |
| Methylpropandiol | 2,00 |
| Allantoin | 0,50 |
| Phenoxyethanol | 0,50 |
| Methylparaben | 0,30 |
| Propylparaben | 0,20 |
| Füllstoffe/ Additive (Iminodisuccinat, Talkum, | 0,50 |
| Distärkephosphat ) | |
| Parfum | q.s. |
| Wasser | 100,00 |
| **PH = 6.5** | |

### (2) O/W Emulsion

| | Gew.-% |
|---|---|
| Glycerylstearatcitrat | 4,00 |
| Jojobaöl | 2,00 |
| Squalan | 1,00 |
| Dicaprylylcarbonat | 2,00 |
| Vaseline | 1,00 |
| Dimethicon (linear) | 2,00 |
| Carageenan | 0,25 |
| Xanthan Gum | 0,05 |
| VP/Hexadecene Copolymer | 0,30 |
| Ethylhexylmethoxycinnamat | 5,00 |
| Butylmethoxydibenzoylmethan | 2,00 |
| Natrium Ascorbylphosphat | 0,10 |
| EDTA | 0,20 |
| Tocopherylacetat | 0,50 |
| Sericoside | 0,20 |
| Glycerin | 6,00 |
| Ethylhexylglycerin | 0,50 |
| Phenoxyethanol | 0,50 |
| Methylparaben | 0,30 |
| Propylparaben | 0,20 |
| lodopropynylbutylcarbamat | 0,02 |
| Füllstoffe/ Additive (Farbstoff, BHT, -Cyclodextrin ) | 0,50 |
| Parfum | q.s. |
| Wasser | 100,00 |
| **PH = 5,5** | |

### (3) O/W Emulsion

| | Gew.-% |
|---|---|
| Glycerylstearatcitrat | 3,00 |
| Macadamiaöl | 1,00 |
| Sqaulan | 2,00 |
| Dicaprylylcarbonat | 1,00 |
| Paraffinöl | 2,00 |
| Dimethicon (linear) | 3,00 |
| Carageenan | 0,30 |
| Xanthan Gum | 0,03 |
| PVP Hexadecen Copolymer | 0,20 |
| Ethylhexylmethoxycinnamat | 3,00 |
| Butylmethoxydibenzoylmethan | 2,00 |
| Ethylhexyltriazon | 1,50 |
| Natrium Ascorbylphosphat | 0,50 |
| EDTA | 0,20 |
| Tocopherylacetat | 0,50 |
| Alpha-Glucosylrutin | 0,05 |
| Glycerin | 6,00 |
| Butylenglycol | 3,00 |
| Phenoxyethanol | 0,50 |
| Methylparaben | 0,30 |
| Propylparaben | 0,20 |
| Butylparaben | 0,05 |
| Füllstoffe/ Additive (Farbstoff, Natrium Citrat) | 0,50 |
| Parfum | q.s. |
| Wasser | 100,00 |
| **PH = 5,0** | |

### (4) O/W Emulsion

| | Gew.-% |
|---|---|
| Glycerylstearatcitrat | 3,00 |
| Caprylic/Capric Triglycerid | 2,00 |
| Squalan | 1,00 |
| Dicaprylylether | 2,00 |
| Dimethylpolysiloxan, cyclisch (Cyclometicone) | 2,00 |
| Isohexadecan | 1,00 |
| Dimethicon (linear) | 1,00 |
| Carbomer | 0,10 |
| Xanthan Gum | 0,20 |
| C18-36 Acid Triglyceride | 0,50 |
| Ethylhexylmethoxycinnamat | 5,00 |
| Bisimidazylate | 1,00 |
| Ethylhexyltriazon | 1,00 |
| Diethylhexyl-butylamidotriazone | 1,00 |
| Natrium Ascorbylphosphat | 0,25 |
| EDTA | 0,20 |
| Tocopherylacetat | 0,50 |
| Traubenkernextrakt, Polyphenol-haltig | 0,25 |
| Kreatin | 0,50 |
| Glycerin | 6,00 |
| Ethylhexylglycerin | 0,50 |
| Phenoxyethanol | 0,50 |
| Methylparaben | 0,30 |
| Propylparaben | 0,20 |
| Butylparaben | 0,05 |
| Füllstoffe/ Additive (Farbstoff, SiO2, Natrium-Citrat) | 0,50 |
| Parfum | q.s. |
| Wasser | 100,00 |
| **PH = 5,5** | |

### 5) Sprühbare O/W Emulsion

| | Gew.-% |
|---|---|
| Glycerylstearatcitrat | 2,00 |
| Caprylic/Capric Triglycerid | 1,50 |
| Myristylmyristat | 1,00 |
| Dicaprylylcarbonat | 1,50 |
| Cyclomethicon | 5,00 |
| Dimethicon (linear) | 2,00 |
| Ethylhexylmethoxycinnamat | 2,00 |
| Bis-Ethylhexyloxyphenol-methoxyphenyltriazine | 0,50 |
| Tocopherylacetat | 0,50 |
| Glycerin | 6,00 |
| Natriumchlorid | 2,50 |
| Iminodisuccinat | 0,20 |
| Xanthan Gum | 0,25 |
| Phenoxyethanol | 0,50 |
| Methylparaben | 0,20 |
| Propylparaben | 0,10 |
| Parfum | q.s. |
| Wasser | 100,00 |
| **PH = 6.0** | |

## Patentansprüche

1. Kosmetische und/oder dermatologische Öl-in-Wasser-(O/W-) Emulsion mit einer Viskosität von 100 bis 1500 mPaS, enthaltend
a) 0,5 bis 8 Gewichts-% eines oder mehrerer Elektrolyten,
b) 0,05 bis 1 Gewichts-% Verdicker,
c) c) 0,5 bis 5 Gewichts-% , Glycerylstearatcitrat
jeweils bezogen auf das Gesamtgewicht der Zubereitung,
wobei der Zubereitung keine Fettalkohole zugesetzt sind.

2. Emulsion nach Anspruch 1, **dadurch gekennzeichnet, dass** Glycerylstearatcitrat in einer Konzentration von 0,5 bis 4 Gewichts-%, bezogen auf das Gesamtgewicht der Zubereitung, eingesetzt wird.

3. Emulsion nach einem der vorhergehenden Ansprüche, **dadurch gekennzeichnet, dass** als Verdicker eine oder mehrere Verbindungen gewählt aus der Gruppe Carrageenan, Xanthangummi, Polyacrylate, Celluloseether, Acryloyldimethyltaurate/VP Copolymer, Hydroxypropyldistärkephosphat (CAS Nummer 113894-92-1).

4. Emulsion nach einem der vorhergehenden Ansprüche, **dadurch gekennzeichnet, dass** als Elektrolyt eine oder mehrere Verbindungen aus der Gruppe der Verbindungen Natriumchlorid, Kaliumchlorid, Magnesiumchlorid, Taurin, Citronensäure, Milchsäure, Ascorbinsäure und ihren Phosphatester, Kreatin, Salicylsäure, Carnitin sowie UV-Filtersubstanzen gewählt aus der Gruppe Phenylbenzimidazolsulfonsäure, Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, insbesondere das Phenylen-1,4-bis-(2-benzimidazyl)-3,3'-5,5'-tetrasulfonsäure-bis-natriumsalz sowie Terephthalidendicamphersulfonsäure oder ihre Salze eingesetzt werden.

5. Emulsion nach einem der vorhergehenden Ansprüche, **dadurch gekennzeichnet, dass** sie UV-Lichtschutzfilter und/oder kosmetische Wirkstoffe enthält.

6. Sprayapplikator in Form eines Pumpsprayapplikators oder einer Aerosoldose enthaltend eine Emulsion nach einem der vorhergehenden Ansprüche.
